# EUROPEAN PATENT APPLICATION

(11) **EP 4 335 451 A1**
(43) Date of publication of application: **13.03.2024**
(21) Application number: 22824797.9
(22) Date of filing: 31.05.2022
(51) Int. Cl.: A61K 38/06, A61K 31/198, A61P 13/12

(54) **PROPHYLACTIC OR THERAPEUTIC AGENT FOR ACUTE RENAL FAILURE**

(30) Priority: 16.06.2021 JP 2021100116
(71) Applicant: Kyowa Hakko Bio Co., Ltd., Tokyo 100-8185 (JP)
(72) Inventor: OHSHIMA Etsuo, Tokyo 100-8185 (JP); MATSUNO Shinya, Tokyo 100-8185 (JP); NISHINO Tsuneyo, Tokyo 100-8185 (JP)
(74) Representative: Vossius & Partner Patentanwälte Rechtsanwälte mbB
(86) International application number: PCT/JP2022/022228
(87) International publication number: WO 2022/264804

(57) **Abstract**

A prophylactic or therapeutic agent for acute renal failure that includes glutathione trisulfide or a pharmacolgically acceptable salt thereof, and a method for determining at least one among the advisability, the method of administration, and the dosage of glutathione trisulfide or a pharmacolgically acceptable salt thereof on the basis of GDF-15 concentration in the blood.

## Description

### Technical Field

The present invention relates to a prophylactic or therapeutic agent for acute renal failure.

### Background Art

Acute renal failure is caused by the rapid and complex involvement of trauma, burns, pancreatitis, hepatitis, thrombosis, sepsis, abnormal cardiopulmonary functions, infectious diseases, inflammatory responses triggered by organ transplantation and drug administration and the like, hypoxia and malnutrition disorders due to ischemic circulation disorders, energy depletion due to mitochondrial dysfunction and the production of tissue-damaging reactive oxygen species. The onset of acute renal failure is feared because it significantly impairs a patient's QOL and exhibits a very high mortality rate. In order to treat acute renal failure, a high-dose steroid therapy for suprresing the production of inflammatory cytokines, a drug therapy such as an anticoagulation therapy for improving systemic peripheral circulation, an acute blood purification therapy for removing pathogenic substances, a continuous renal support therapy, plasma exchange and the like are performed. However, there is still no treatment method for improving patient prognosis, particularly, life prognosis, and the development of a new prophylactic agent/therapeutic agent effective for acute renal failure has become a major medical challenge.

Acute renal failure is thought to be caused by a complex involvement of the above causes, resulting in severe renal function failure. The above causes can be expressed as various types of stress applied to living bodies, and in recent years, growth differentiation factor-15 (GDF-15) has been receiving focus as a stress marker that responds sensitively when various types of stress are applied to living bodies (Non-Patent Literature 3). It has been reported that GDF-15 increases rapidly after ischemia reperfusion in ischemic renal injury (Non-Patent Literature 4). In addition, it has been reported that the blood GDF-15 concentration is a predictive marker for poor prognosis of renal failure (Non-Patent Literature 5).

It has been reported that polysulfides such as glutathione trisulfide (GSSSG) may have physiological functions such as anti-aging (for example, Non-Patent Literature 1 and 2). It has been reported that some polysulfides are involved in mitochondrial functions such as mitochondria energy production (Non-Patent Literature 6). In addition, as a method of producing a trisulfide compound, the method described in Patent Literature 1 and the like are known.

### Citation List

### Patent Literature

[Patent Literature 1] WO 2018/117186

### Non-Patent Literature

[Non-Patent Literature 1] T. Ida ,et al. , PNAS, May 27, 2014, 111(21) 7606-7611
[Non-Patent Literature 2] T. Akaike et al., Nature Communications, 2017, Oct27; 8(1): 1177
[Non-Patent Literature 3] Y. Fujita et al., Geriatr. Gerontol. Int, 2016, Mar; 16,Suppl,1: 17-29.
[Non-Patent Literature 4] J. Liu et al., J. Am. Soc. Nephrol., 2020, Apr; 31(4): 701-715.
[Non-Patent Literature 5] J-H. Lim et al., J. Clin. Med., 2021, Aug 18; 10(16): 3660.
[Non-Patent Literature 6] S. Fujii et al., Br. J. Pharmacol., 2019, Feb; 176(4): 607-615.

### Summary of Invention

### Technical Problem

An object of the present invention is to provide a prophylactic or therapeutic agent for acute renal failure, which has a prophylactic effect or therapeutic effect.

### Solution to Problem

The inventors evaluated the effects of glutathione trisulfide in acute renal failure model animals. As a result, they found that increases in the blood urea nitrogen (UN) concentration and blood creatinine (CRE) concentration were suppressed in the administration group, and completed the present invention. In addition, they found that measurement of the blood GDF-15 concentration may be useful in selecting whether or not to administer glutathione trisulfide, a usage method and/or a dosage, and completed the present invention.

The present invention provides the following [1] to [12].
[1] A prophylactic or therapeutic agent for acute renal failure, comprising glutathione trisulfide or a pharmacologically acceptable salt thereof.
[2] A method of prevent or treat an acute renal failure, comprising administering glutathione trisulfide or a pharmacologically acceptable salt thereof to a patient in need thereof.
[3] Glutathione trisulfide or a pharmacologically acceptable salt thereof for use in preventing or treating acute renal failure.
[4] Use of glutathione trisulfide or a pharmacologically acceptable salt thereof for the manufacture of a prophylactic or therapeutic agent for acute renal failure.
[5] A method of predicting or determining whether or not to administer glutathione trisulfide or a pharmacologically acceptable salt thereof, a usage method or a dosage to a subject, comprising steps of
   measuring a blood GDF-15 concentration in a subject; and
   predicting or determining at least one of whether or not to administer glutathione trisulfide or a pharmacologically acceptable salt thereof, a usage method and a dosage to the subject based on the blood GDF-15 concentration.
[6] The method according to [5],
   wherein the prediction or determination comprises estimating a blood urea nitrogen concentration and/or a blood creatinine concentration in the subject based on the blood GDF-15 concentration, and predicting or determining at least one of whether or not to administer glutathione trisulfide or a pharmacologically acceptable salt thereof, a usage method and a dosage to the subject based on the blood urea nitrogen concentration and/or the blood creatinine concentration.
[7] A method of predicting or determining whether or not to administer glutathione trisulfide or a pharmacologically acceptable salt thereof, a usage method or a dosage to a patient with acute renal failure, comprising steps of
   measuring a blood GDF-15 concentration in a patient with acute renal failure and
   predicting or determining at least one of whether or not to administer glutathione trisulfide or a pharmacologically acceptable salt thereof, a usage method and a dose to the patient with acute renal failure based on the blood GDF-15 concentration.
[8] A method of preparing a prophylactic or therapeutic agent for acute renal failure comprising glutathione trisulfide or a pharmacologically acceptable salt thereof, comprising steps of
   measuring a blood GDF-15 concentration in a subject; and
   predicting or determining a content of glutathione trisulfide or a pharmacologically acceptable salt thereof in the prophylactic or therapeutic agent based on the blood GDF-15 concentration.
[9] A method of measuring an indicator for prognosis determination and/or diagnosis of acute renal failure *in vitro,* comprising
   measuring a GDF-15 concentration for a sample obtained from a subject as the indicator.
[10] A method of predicting or determining at least one of whether or not to administer glutathione trisulfide or a pharmacologically acceptable salt thereof, a usage method and a dosage to a patient with acute renal failure, comprising
   measuring a blood GDF-15 concentration in the patient with acute renal failure.
[11] A method of providing a blood GDF-15 concentration in a patient with acute renal failure for predicting or determining at least one of whether or not to administer glutathione trisulfide or a pharmacologically acceptable salt thereof, a usage method and a dosage to the patient with acute renal failure, comprising measuring a blood GDF-15 concentration in the patient with acute renal failure.
[12] The prophylactic or therapeutic agent for acute renal failure, the preventing or treating method, the glutathione trisulfide or the pharmacologically acceptable salt thereof for use, the use, or the method according to any one of [1] to [4], and [7] to [11],
   wherein the acute renal failure is acute renal failure resulting from at least one disease, disorder or condition selected from the group consisting of myoglobinuria rhabdomyolysis, hemoglobinuria, atheroembolism, renal artery occlusion, vasculitis, hemolytic uremic syndrome, thrombolytic thrombocytopenic purpura, Wegener's granulomatosis, systemic lupus erythematosus, Henoch-Schoenlein purpura, Goodpasture's syndrome, acute glomerulonephritis, acute hypersensitivity intestinal nephritis, hypercalcemia, urinary tract obstruction, acute uric acid nephropathy, pyelonephritis, papillary necrosis, hepatorenal syndrome, hypertension, bacterial sepsis, systemic inflammatory shock, fungemia, acute viral diseases, severe body fluid volume depletion, burns, ischemia reperfusion, postpartum complications, surgical intervention, and drug nephritis, and is associated with renal ischemia or renal ischemia reperfusion.

### Advantageous Effects of Invention

According to the present invention, it is possible to provide a prophylactic or therapeutic agent for acute renal failure, which has a prophylactic effect or therapeutic effect. In addition, it is possible to provide a method of selecting whether or not to administer glutathione trisulfide or a pharmacologically acceptable salt thereof, a usage method and a dosage using a blood growth differentiation factor-15 (GDF-15) concentration as an indicator when preventing or treating acute renal failure.

### Brief Description of Drawings

FIG. 1 is a graph showing the change in a blood urea nitrogen concentration in a rat ischemic acute renal injury model with unilateral nephrectomy.
FIG. 2 is a graph showing the change in a blood creatinine concentration in a rat ischemic acute renal injury model with unilateral nephrectomy.
FIG. 3(a) is a graph showing the correlation between a blood urea nitrogen concentration and a GDF-15 value in a rat bilateral renal simultaneous ligation ischemic acute renal injury model, and FIG. 3(b) is a graph showing the correlation between the blood creatinine concentration and the GDF-15 value in the rat bilateral renal simultaneous ligation ischemic acute renal injury model.
FIG. 4(a) is a graph showing the correlation between the blood urea nitrogen concentration and the GDF-15 value in the rat bilateral renal simultaneous ligation ischemic acute renal injury model, and FIG. 4(b) is a graph showing the correlation between the blood creatinine concentration and the GDF-15 value in the rat bilateral renal simultaneous ligation ischemic acute renal injury model.

### Description of Embodiments

Hereinafter, the content of the present invention will be described in detail, but the present invention is not limited to the following embodiments.

A prophylactic or therapeutic agent and a preventing or treating method of the present invention, and other methods described in this specification may be administered or applied to humans.

One embodiment of the present invention relates to a prophylactic or therapeutic agent for acute renal failure, comprising glutathione trisulfide or a pharmacologically acceptable salt thereof.

In the present invention, examples of a pharmacologically acceptable salt include salts of inorganic acids such as hydrochloric acid, hydrobromic acid, sulfuric acid, nitric acid, and phosphoric acid; salts of organic acids such as acetic acid, succinic acid, fumaric acid, maleic acid, tartaric acid, citric acid, lactic acid, stearic acid, benzoic acid, methanesulfonic acid, ethanesulfonic acid, and p-toluenesulfonic acid; salts of alkali metals such as sodium and potassium; salts of alkaline earth metals such as calcium and magnesium; ammonium salts; and salts of amino acids such as arginine.

In the present invention, glutathione trisulfide or apharmacologically acceptable salt thereof may have crystal polymorphs, but the present invention is not limited thereto, and it may be a single substance of any crystal form or a mixture. In addition, glutathione trisulfide or a pharmacologically acceptable salt thereof in the present invention also includes an amorphous component. Furthermore, glutathione trisulfide or a pharmacologically acceptable salt thereof in the present invention includes anhydrides and solvates (particularly, hydrates).

For example, the prophylactic or therapeutic agent for acute renal failure according to one embodiment of the present invention can be administered orally in the form of tablets, capsules, powders, granules, liquid formulations or syrup agents, or administered non-orally in the form of injections, infusions, or suppositories. It can be formulated into these dosage forms using known formulation techniques. In the case of solid agents, pharmacologically acceptable excipients, for example, starch, lactose, refined white sugar, glucose, crystalline cellulose, carboxycellulose, carboxymethyl cellulose, carboxyethyl cellulose, calcium phosphate, magnesium stearate, and gum arabic, can be added during formulation, and if necessary, lubricants, binders, disintegrants, coating agents, colorants and the like can be added. In addition, in the case of liquid formulations, stabilizers, dissolution aids, suspending agents, emulsifiers, buffering agents, preservatives and the like can be added.

The dosage of the prophylactic or therapeutic agent for acute renal failure of the present invention is a therapeutically effective amount, which varies depending on the symptoms, age, administration method, dosage form and the like, but in general cases, 0.5 to 2,000 mg of the compound per day can be administered to adults, and it is preferable to administer 1 to 800 mg once per day or in divided doses over consecutive days. It is thought that, if the dosage of glutathione trisulfide or a pharmacologically acceptable salt thereof is within the range, a prophylactic effect or therapeutic effect on acute renal failure is exhibited.

One embodiment of the present invention relates to a method of predicting or determining whether or not to administer glutathione trisulfide or a pharmacologically acceptable salt thereof, a usage method or a dosage to a subject, including the steps of measuring a blood GDF-15 concentration in a subject and predicting or determining at least one of whether or not to administer glutathione trisulfide or a pharmacologically acceptable salt thereof, a usage method and a dosage to the subject based on the blood GDF-15 concentration.

The predicting or determining may include estimating a blood urea nitrogen concentration and/or a blood creatinine concentration in the subject based on the blood GDF-15 concentration, and predicting or determining at least one of whether or not to administer glutathione trisulfide or a pharmacologically acceptable salt thereof, a usage method and a dosage to the subject based on the blood urea nitrogen concentration and/or the blood creatinine concentration.

One embodiment of the present invention relates to a method of preparing a prophylactic or therapeutic agent for acute renal failure comprising glutathione trisulfide or a pharmacologically acceptable salt thereof, including the steps of measuring a blood GDF-15 concentration in a subject, and predicting or determining a content of glutathione trisulfide or a pharmacologically acceptable salt thereof in the prophylactic or therapeutic agent based on the blood GDF-15 concentration.

The method of preparing a prophylactic or therapeutic agent may include predicting or determining by the subject the content of glutathione trisulfide or a pharmacologically acceptable salt thereof in the prophylactic or therapeutic agent. The prophylactic or therapeutic agent is not limited to a single formulation, and may be a combination of a plurality of formulations. When the prophylactic or therapeutic agent is a single formulation, the subject can select a formulation comprising an appropriate amount of glutathione trisulfide or a pharmacologically acceptable salt thereof based on the measured value of GDF-15. When the prophylactic or therapeutic agent is a combination of a plurality of formulations, by changing the combination of the plurality of formulations, the subject him/herself can predict or determine the content of glutathione trisulfide or a pharmacologically acceptable salt thereof in the prophylactic or therapeutic agent.

One embodiment of the present invention relates to a method of measuring an indicator for prognosis determination and/or diagnosis of acute renal failure *in vitro*, including measuring a GDF-15 concentration for a sample obtained from a subject as the indicator. The method according to the present embodiment may further include the steps of estimating a blood urea nitrogen concentration and/or a blood creatinine concentration in the subject based on the blood GDF-15 concentration, and predicting or determining at least one of whether or not to administer glutathione trisulfide or a pharmacologically acceptable salt thereof, a usage method and a dosage to the subject based on the blood urea nitrogen concentration and/or the blood creatinine concentration.

One embodiment of the present invention relates to a method of predicting or determining at least one of whether or not to administer glutathione trisulfide or a pharmacologically acceptable salt thereof, a usage method and a dosage to a patient with acute renal failure, including measuring a blood GDF-15 concentration in the patient with acute renal failure. The method according to the present embodiment may further include the steps of estimating a blood urea nitrogen concentration and/or a blood creatinine concentration in the subject based on the blood GDF-15 concentration, and predicting or determining at least one of whether or not to administer glutathione trisulfide or a pharmacologically acceptable salt thereof, a usage method and a dosage to the subject based on the blood urea nitrogen concentration and/or the blood creatinine concentration.

One embodiment of the present invention relates to a method of providing a blood GDF-15 concentration in a patient with acute renal failure for predicting or determining at least one of whether or not to administer glutathione trisulfide or a pharmacologically acceptable salt thereof, a usage method and a dosage to the patient with acute renal failure, including measuring a blood GDF-15 concentration in the patient with acute renal failure. The method according to the present embodiment may further include the steps of estimating a blood urea nitrogen concentration and/or a blood creatinine concentration in the subject based on the blood GDF-15 concentration, and predicting or determining at least one of whether or not to administer glutathione trisulfide or a pharmacologically acceptable salt thereof, a usage method and a dosage to the subject based on the blood urea nitrogen concentration and/or the blood creatinine concentration.

In this specification, "subject" may be a patient diagnosed with acute renal failure or a person before being diagnosed with acute renal failure. In this specification, "subject" may be a person with chronic diseases such as hypertension and diabetes.

The blood GDF-15 concentration, which is a reference for predicting or determining at least one of whether or not to administer glutathione trisulfide or a pharmacologically acceptable salt thereof, a usage method and a dosage, varies depending on the symptoms, age, administration method, dosage form and the like, but for normal adults, the concentration is, for example, 0.05 ng/mL to 100 ng/mL, and more preferably 0.1 ng/mL to 10 ng/mL. If the blood GDF-15 concentration is within this range, it can be determined that it is better to administer glutathione trisulfide or a pharmacologically acceptable salt thereof, and in this case, the usage method and/or dosage described in this specification can be applied.

Examples of acute renal failure in the present invention include at least one disease, disorder or condition selected from the group consisting of myoglobinuria rhabdomyolysis, hemoglobinuria, atheroembolism, renal artery occlusion, vasculitis, hemolytic uremic syndrome, thrombolytic thrombocytopenic purpura, Wegener's granulomatosis, systemic lupus erythematosus, Henoch-Schoenlein purpura, Goodpasture's syndrome, acute glomerulonephritis, acute hypersensitivity intestinal nephritis, hypercalcemia, urinary tract obstruction, acute uric acid nephropathy, pyelonephritis, papillary necrosis, hepatorenal syndrome, hypertension, bacterial sepsis, systemic inflammatory shock, fungemia, acute viral diseases, severe body fluid volume depletion, burns, ischemia reperfusion, postpartum complications, surgical intervention, and drug nephritis, and is associated with renal ischemia or renal ischemia reperfusion.

### [Examples]

Hereinafter, the present invention will be described in more detail with reference to examples, but the present invention is not limited thereto.

### <Example 1: evaluation of effect of glutathione trisulfide (GSSSG) in rat ischemic acute renal injury model with unilateral nephrectomy>

GSSSG dihydrate produced by the method described in Patent Literature 1 was carefully crushed in a mortar for 10 minutes or longer. 1,333 mg (1,250 mg in terms of anhydride) of GSSSG dihydrate was weighed out. 1,333 mg of the crushed GSSSG dihydrate was added to 40 mL of a normal saline, and the mixture was stirred in a suspended state for 5 minutes. 0.25 to 0.50 g of sodium bicarbonate was added to the suspension and dissolved. This GSSSG solution was transferred to a measuring cylinder, and a normal saline was added to make up 50 mL to obtain a preparation solution (final concentration: 25 mg/mL). The obtained preparation solution was filtered with a filter (0.22 µm filter) and used as an administration sample.

In the test, 4-week old male SD rats (body weight 99 to 113 g) were used. A 16-day preliminary breeding period was set for the obtained animals. Rats with no abnormalities in body weight change and general condition were used for grouping.

One kidney was removed from rats in which no abnormalities were observed after the preliminary breeding period by the following method. The rats were fixed in the left lateral position under isoflurane anesthesia, and the hair on the right lower back was shaved. After analgesic treatment with subcutaneous administration of 1 mg/kg (1 mL/kg) of butorphanol tartrate, the right lower back was incised, the right kidney was removed, and the renal arteries and veins were ligated and then removed. After the right kidney was removed, the incision was sutured.

The administration sample was administered into the peritoneal cavity of rats from which the right kidney was removed using a disposable polypropylene syringe with an injection needle. The day on which the ischemia treatment was performed was defined as day0, and during the 4-day period from 4 days before (day (-4)) the ischemia treatment was performed to 1 day before (day (-1)) the ischemia treatment was performed, the sample was administered once a day, a total of 4 times. The amount of solution administered was calculated at 8 mL/kg based on the body weight on the first day of administration. Here, the same amount of a normal saline was administered to the placebo surgery group and the control group.

Table 1 shows the group composition, the dosage of GSSSG and the number of animals used.

**[Table 1]**

| Group number | Group name | Dosage of GSSSG (mg/kg) | Number of animals (animal number) |
|---|---|---|---|
| 1 | Placebo surgery | - | 4 |
| 2 | Control | - | 8 |
| 3 | GSSSG | 200 | 8 |

The ischemia and reperfusion treatments were performed as follows. Anesthesia was performed by intraperitoneal administration of 45 mg/kg (1 mL/kg) of pentobarbital sodium and subcutaneous administration of 1 mg/kg (1 mL/kg) of butorphanol tartrate. The left dorsal part where the kidney was located was shaved. Then, the animals were treated on a heater mat. The surgical site was disinfected with about 70% of an alcohol and then incised using scissors. After the left kidney was exposed, the renal arteries and veins were clamped using Klemme (clip power 120 g) for 40 minutes. During clamping, the kidney was returned to the body in order to prevent kidney desiccation. During reperfusion, it was confirmed that the blood color of the kidney was returned to normal (red), and the laparotomy wound was closed and disinfected with Isodine. The left kidney of the rats in the placebo surgery group was exposed, and returned to the peritoneal cavity without ischemia treatment, and the laparotomy wound was closed and disinfected with Isodine 40 minutes after incision. After suturing, the rats were returned to the original breeding cage.

On the day of unilateral nephrectomy (before removal), the day before administration started, the day of last administration (4 hours or longer after administration), and 24 and 48 hours after the treatment was completed [in the control group, and the GSSSG group, the time when the left kidney was removed (reperfusion) using Klemme was defined as the time at which the treatment was completed, and in the placebo surgery group, the time when the abdomen was closed was defined as the time when the treatment was completed], without anesthesia, about 0.25 mL of blood was collected from the tail vein using an EDTA-2K-treated capillary. 72 hours after the treatment, under isoflurane anesthesia, all blood was collected from the abdominal aorta using a winged injection needle and an EDTA-2K-treated vacuum blood collection tube. The allowable range of the blood sampling time was ±1 hour. The collected blood (EDTA-2K added) was cooled on ice and then separated using a centrifuge (4°C, 2,200×g, for 15 minutes). Blood 72 hours after the treatment was separated using a centrifuge (4°C, 2,150×g, for 15 minutes). The urea nitrogen (UN) and creatinine (CRE) concentrations were measured using the obtained plasma. Test items and measurement methods are as shown in Table 2.

**[Table 2]**

| Item | Unit | Measurement method |
|---|---|---|
| Urea nitrogen (UN) | mg/dL | Urease/GLDH method (ammonia elimination method) |
| Creatinine (CRE) | mg/dL | Creatininase/HMMPS method |

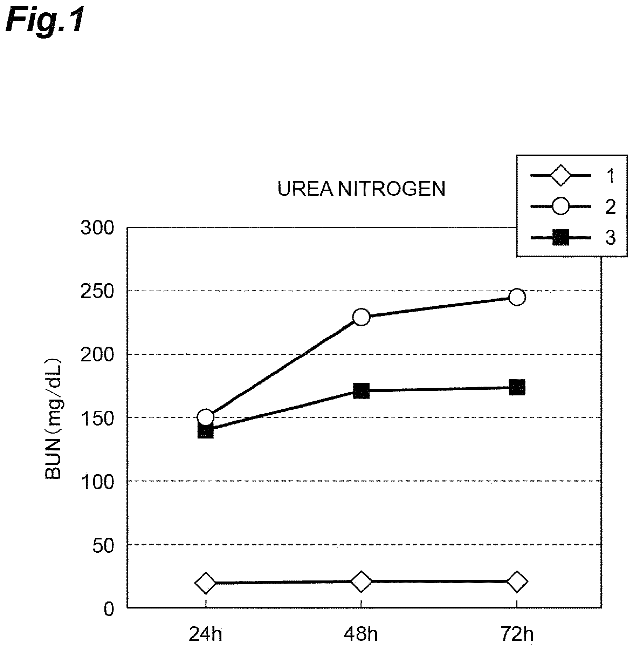

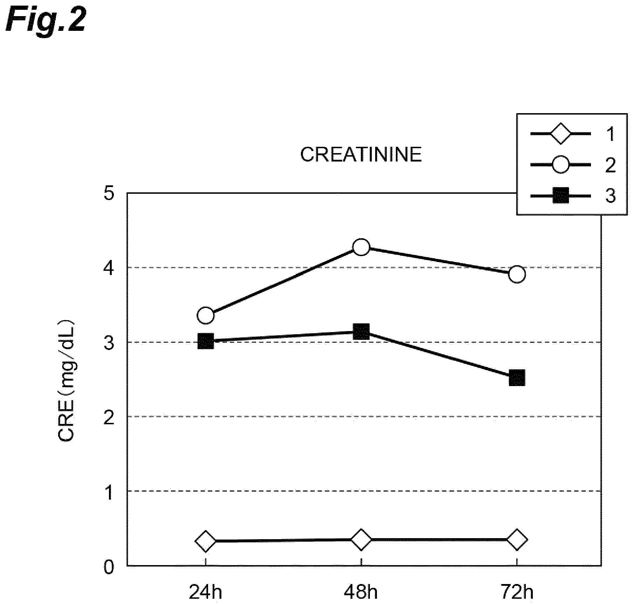

When the UN and CRE concentrations in plasma were measured according to the grouping shown Table 1, the measured values were higher in the control group in which ischemia and reperfusion were performed than in the placebo surgery group. On the other hand, in the 200 mg/kg GSSSG administration group, the UN value (FIG. 1) and the CRE value (FIG. 2) decreased.

### <Example 2: evaluation of effect of GSSSG in rat bilateral kidney simultaneous ligation ischemic acute renal injury model>

GSSSG dihydrate produced by the method described in Patent Literature 1 was carefully crushed in a mortar for 10 minutes or longer. A required amount of GSSSG dihydrate was weighed out. 0.5 w/v% methylcellulose (hereinafter abbreviated to as MC) was added to the crushed GSSSG dihydrate to prepare a suspension at a concentration of 10 mg/mL, and used as an administration sample for a high-dose administration group. The 10 mg/mL suspension was diluted with 0.5 w/v% MC to prepare a 4 mg/mL suspension, and used as an administration sample for a low-dose administration group.

In the test, 5-week old male SD rats (body weight 136 to 154 g) were used. An 8-day preliminary breeding period was set for the obtained animals. Rats with no abnormalities in body weight change and general condition were used for grouping.

The administration method was oral administration. Forced oral administration was performed using a disposable polypropylene syringe with a metal oral gavage tube for rats.

The amount of solution administered was 5 mL/kg for both the high dose group and the low dose group based on the body weight closest to that of the day of administration. Administration was performed a total of 5 times: 1 day before, 2 days before, and 3 days before the ischemia treatment was performed, 1 hour before the ischemia treatment, and 2 hours after the reperfusion treatment.

Table 3 shows the group composition, the dosage of GSSSG and the number of animals used.

**[Table 3]**

| Group number | Group name | Dosage (mg/kg) | Number of animals |
|---|---|---|---|
| 1 | Placebo surgery | - | 4 |
| 2 | Control | - | 8 |
| 3 | GSSSG | 20 | 8 |
| 4 | GSSSG | 50 | 8 |

The ischemia and reperfusion treatments were performed as follows. Anesthesia was performed by intraperitoneal administration of 45 mg/kg (1mL/kg) of pentobarbital sodium and subcutaneous administration of 1 mg/kg (1 mL/kg) of butorphanol tartrate. Both dorsal parts where the kidneys were located were shaved. Then, the animals were treated on a heater mat. The surgical site was disinfected with about 70% of an alcohol and then incised using scissors. After both kidneys of the animals in Group 2 to Group 4 in Table 3 were exposed, the renal arteries and veins of the left kidney and the right kidney in order were clamped using Klemme (clip power 120 g) for 40 minutes. During clamping, the kidney was returned to the body in order to prevent kidney desiccation. During reperfusion, it was confirmed that the blood color of the kidney was returned to normal (red), and the laparotomy wound was closed and disinfected with Isodine. In the animals of the Group 1, both kidneys were exposed, and returned to the peritoneal cavity without ischemia treatment, and the laparotomy wound was closed and disinfected with Isodine 40 minutes after incision. After suturing, the rats were returned to the original breeding cage.

On the day before administration, without anesthesia, about 0.25 mL of blood was collected from the tail vein using a heparin Na-treated capillary (DRUMMOND). The collected blood was cooled on ice and then separated using a centrifuge (4°C, 2,200×g, for 15 minutes), and the UN concentration and the CRE concentration were measured using the obtained plasma.

48 hours after the reperfusion treatment, under isoflurane anesthesia, all blood was collected from abdominal aorta, using a winged injection needle and a heparin Na-treated vacuum blood collection tube. The collected blood was cooled on ice and then separated using a centrifuge (4°C, 2,150×g, for 15 minutes), and UN, CRE and GDF-15 (Mouse/Rat GDF-15 Quantikine ELISA Kit, R&D Systems, Inc) were measured using the obtained plasma. Test items and measurement methods are as shown in Table 4.

**[Table 4]**

| Item | Unit | Measurement method |
|---|---|---|
| Urea nitrogen (UN) | mg/dL | Urease/GlDH method (ammonia elimination method) |
| Creatinine (CRE) | mg/dL | Creatininase/HMMPS method |
| GDF-15 | pg/mL | ELISA method |

When the UN and CRE concentrations in plasma were measured according to the grouping shown in Table 3, the measured values were higher in the control group in which ischemia and reperfusion were performed than in the placebo surgery group. On the other hand, in the GSSSG administration group, the UN value (Table 5) and the CRE value (Table 6) decreased in a dose-dependent manner. In addition, the GDF-15 value (Table 7) was higher in the control group in which ischemia and reperfusion were performed than in the placebo surgery group.

**[Table 5]**

| Blood urea nitrogen (UN) | Before administration mg/dL | 48 hours after ischemia reperfusion mg/dL |
|---|---|---|
| Placebo surgery | 12.8±0.9 | 14.4±1.1 |
| Control | 12.8±0.6 | 154.6±19.5 |
| 20 mg/kg | 13.5±0.5 | 136.8±24.6 |
| 50 mg/kg | 11.5±0.4 | 106.2±16.5 |

**[Table 6]**

| Blood creatinine (CRE) | Before administration mg/dL | 48 hours after ischemia reperfusion mg/dL |
|---|---|---|
| Placebo surgery | 0.22±0.00 | 0.24±0.01 |
| Control | 0.21±0.00 | 2.98±0.56 |
| 20 mg/kg | 0.21±0.00 | 2.29±0.52 |
| 50 mg/kg | 0.20±0.00 | 2.07±0.45 |

**[Table 7]**

| GDF-15 (growth differentiation factor) | 48 hours after ischemia reperfusion pg/mL |
|---|---|
| Placebo surgery | 75.2±4.3 |
| Control | 1532±246 |
| 20 mg/kg | 1673±595 |
| 50 mg/kg | 1327±300 |

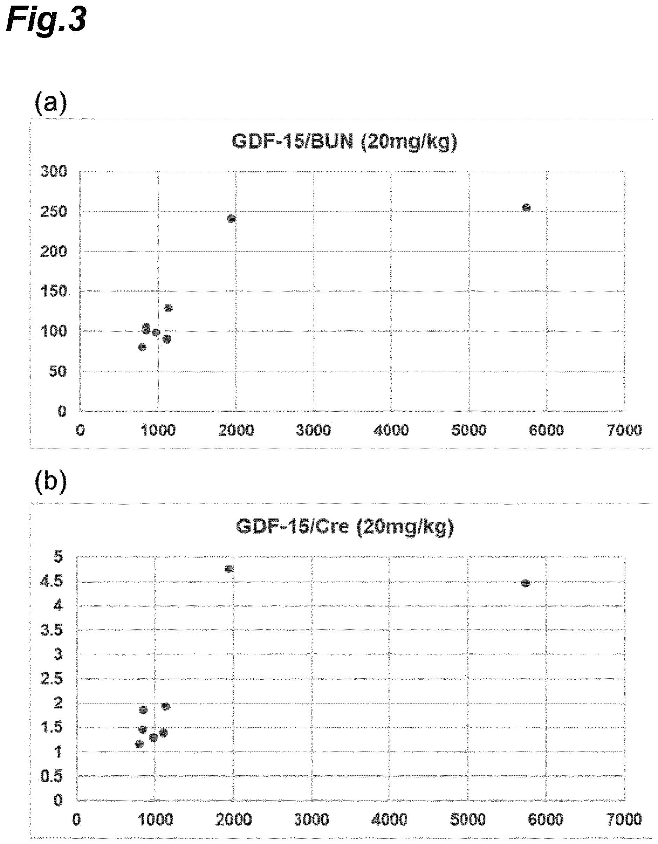

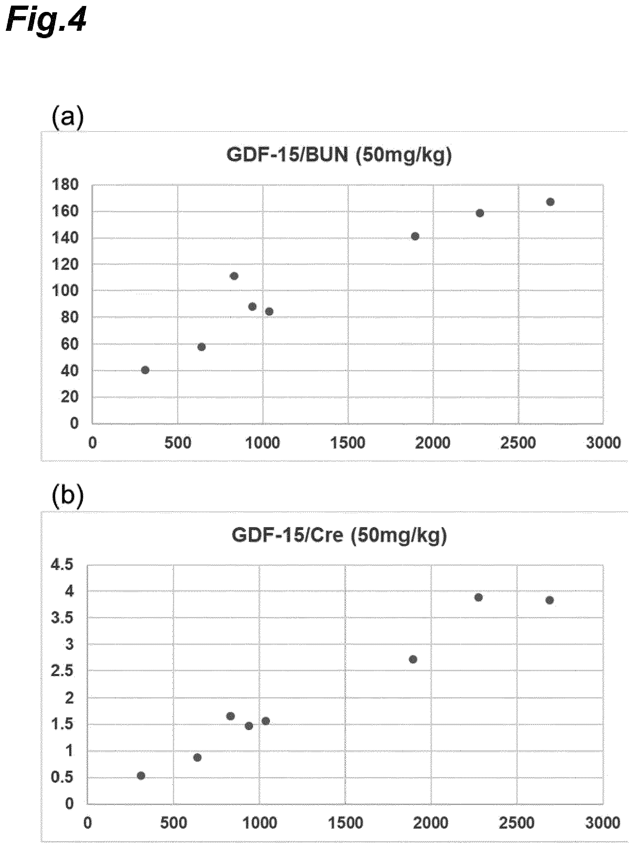

On the other hand, in the GSSSG administration group, the GDF-15 concentration slightly decreased in the 50 mg/kg group. In the 20 mg/kg administration group and 50 mg/kg group in Table 7, the correlation between the UN concentration and the CRE concentration, which are renal function indicators, and the GDF-15 concentration was examined. The results are shown in FIGS. 3 and 4. The correlation coefficient between the UN concentration and theGDF-15 concentration in the 20 mg/kg administration group was 0.82, indicating that there was a correlation between the two (FIG. 3(a)). The correlation coefficient between the CRE concentration and the GDF-15 concentration in the 20 mg/kg administration group was 0.75, indicating that there was a correlation between the two (FIG. 3(b)). In the 50 mg/kg administration group in which a therapeutic effect was exhibited, the correlation coefficient between the UN concentration and the GDF-15 concentration was 0.95, indicating that there was a high correlation between the two (FIG. 4(a)). In the 50 mg/kg group in which a therapeutic effect was exhibited, the correlation coefficient between the CRE concentration and the GDF-15 concentration was 0.98, indicating that there was a high correlation between the two (FIG. 4(b)). These results showed that the severity of renal dysfunction and the therapeutic effect of GSSSG, and the GDF-15 concentration were correlated.

When renal functions decreased or worsened, the blood GDF-15 concentration increased more quickly than the blood UN concentration and the blood CRE concentration, which have been conventionally used as renal function indicators. In addition, the increase in the blood GDF-15 concentration was larger than the increase in the blood UN concentration and the blood CRE concentration. This and the results of this example suggested that the blood GDF-15 concentration could be used as a more preferable marker for renal diseases. In addition, this example showed a correlation between the therapeutic effect of GSSSG and the GDF-15 concentration. This suggested that the GDF-15 concentration could be used as an indicator for predicting or determining the GSSSG dosage and the like.

FIGS. 3 and 4 suggest that the blood GDF-15 concentration may be correlated with organ dysfunction due to ischemia reperfusion and may be correlated with drug effects. This suggests a possibility of grasping the correlation with organ dysfunction and the effects of drugs using GDF-15 as an indicator even in organs in which clear indicators of organ dysfunction have not been defined such as the kidney. More specifically, it suggests a possibility when polysulfides were used as drugs, more specifically, when glutathione trisulfide was used as a drug. Organs damaged by ischemia reperfusion include the brain, spinal cord, lungs, sensory nerves, motor nerves, heart, and liver addition to the kidney, but the present invention is not limited thereto.

## Claims

1. A prophylactic or therapeutic agent for acute renal failure, comprising glutathione trisulfide or a pharmacologically acceptable salt thereof.

2. The prophylactic or therapeutic agent according to claim 1,
wherein the acute renal failure is acute renal failure resulting from at least one disease, disorder or condition selected from the group consisting of myoglobinuria rhabdomyolysis, hemoglobinuria, atheroembolism, renal artery occlusion, vasculitis, hemolytic uremic syndrome, thrombolytic thrombocytopenic purpura, Wegener's granulomatosis, systemic lupus erythematosus, Henoch-Schoenlein purpura, Goodpasture's syndrome, acute glomerulonephritis, acute hypersensitivity intestinal nephritis, hypercalcemia, urinary tract obstruction, acute uric acid nephropathy, pyelonephritis, papillary necrosis, hepatorenal syndrome, hypertension, bacterial sepsis, systemic inflammatory shock, fungemia, acute viral diseases, severe body fluid volume depletion, burns, ischemia reperfusion, postpartum complications, surgical intervention, and drug nephritis, and is associated with renal ischemia or renal ischemia reperfusion.

3. A method of prevent or treat an acute renal failure, comprising
administering glutathione trisulfide or a pharmacologically acceptable salt thereof to a subject in need thereof.

4. The preventing or treating method according to claim 3,
wherein the acute renal failure is acute renal failure resulting from at least one disease, disorder or condition selected from the group consisting of myoglobinuria rhabdomyolysis, hemoglobinuria, atheroembolism, renal artery occlusion, vasculitis, hemolytic uremic syndrome, thrombolytic thrombocytopenic purpura, Wegener's granulomatosis, systemic lupus erythematosus, Henoch-Schoenlein purpura, Goodpasture's syndrome, acute glomerulonephritis, acute hypersensitivity intestinal nephritis, hypercalcemia, urinary tract obstruction, acute uric acid nephropathy, pyelonephritis, papillary necrosis, hepatorenal syndrome, hypertension, bacterial sepsis, systemic inflammatory shock, fungemia, acute viral diseases, severe body fluid volume depletion, burns, ischemia reperfusion, postpartum complications, surgical intervention, and drug nephritis, and is associated with renal ischemia or renal ischemia reperfusion.

5. Glutathione trisulfide or a pharmacologically acceptable salt thereof for use in preventing or treating acute renal failure.

6. The glutathione trisulfide or the pharmacologically acceptable salt thereof for use according to claim 5,
wherein the acute renal failure is acute renal failure resulting from at least one disease, disorder or condition selected from the group consisting of myoglobinuria rhabdomyolysis, hemoglobinuria, atheroembolism, renal artery occlusion, vasculitis, hemolytic uremic syndrome, thrombolytic thrombocytopenic purpura, Wegener's granulomatosis, systemic lupus erythematosus, Henoch-Schoenlein purpura, Goodpasture's syndrome, acute glomerulonephritis, acute hypersensitivity intestinal nephritis, hypercalcemia, urinary tract obstruction, acute uric acid nephropathy, pyelonephritis, papillary necrosis, hepatorenal syndrome, hypertension, bacterial sepsis, systemic inflammatory shock, fungemia, acute viral diseases, severe body fluid volume depletion, burns, ischemia reperfusion, postpartum complications, surgical intervention, and drug nephritis, and is associated with renal ischemia or renal ischemia reperfusion.

7. Use of glutathione trisulfide or a pharmacologically acceptable salt thereof for the manufacture of a prophylactic or therapeutic agent for acute renal failure.

8. The use according to claim 7,
wherein the acute renal failure is acute renal failure resulting from at least one disease, disorder or condition selected from the group consisting of myoglobinuria rhabdomyolysis, hemoglobinuria, atheroembolism, renal artery occlusion, vasculitis, hemolytic uremic syndrome, thrombolytic thrombocytopenic purpura, Wegener's granulomatosis, systemic lupus erythematosus, Henoch-Schoenlein purpura, Goodpasture's syndrome, acute glomerulonephritis, acute hypersensitivity intestinal nephritis, hypercalcemia, urinary tract obstruction, acute uric acid nephropathy, pyelonephritis, papillary necrosis, hepatorenal syndrome, hypertension, bacterial sepsis, systemic inflammatory shock, fungemia, acute viral diseases, severe body fluid volume depletion, burns, ischemia reperfusion, postpartum complications, surgical intervention, and drug nephritis, and is associated with renal ischemia or renal ischemia reperfusion.

9. A method of determining whether or not to administer glutathione trisulfide or a pharmacologically acceptable salt thereof, a usage method or a dosage to a subject, comprising steps of:
measuring a blood GDF-15 concentration in a subject; and
determining at least one of whether or not to administer glutathione trisulfide or a pharmacologically acceptable salt thereof, a usage method and a dosage to the subject based on the blood GDF-15 concentration.

10. The method according to claim 9,
wherein the determination comprises:
estimating a blood urea nitrogen concentration and/or a blood creatinine concentration in the subject based on the blood GDF-15 concentration, and
determining at least one of whether or not to administer glutathione trisulfide or a pharmacologically acceptable salt thereof, a usage method and a dosage to the subject based on the blood urea nitrogen concentration and/or the blood creatinine concentration.

11. A method of determining whether or not to administer glutathione trisulfide or a pharmacologically acceptable salt thereof, a usage method or a dosage to a patient with acute renal failure, comprising steps of:
measuring a blood GDF-15 concentration in a patient with acute renal failure; and
determining at least one of whether or not to administer glutathione trisulfide or a pharmacologically acceptable salt thereof, a usage method and a dosage to the patient with acute renal failure based on the blood GDF-15 concentration.

12. The method according to claim 11,
wherein the acute renal failure is acute renal failure resulting from at least one disease, disorder or condition selected from the group consisting of myoglobinuria rhabdomyolysis, hemoglobinuria, atheroembolism, renal artery occlusion, vasculitis, hemolytic uremic syndrome, thrombolytic thrombocytopenic purpura, Wegener's granulomatosis, systemic lupus erythematosus, Henoch-Schoenlein purpura, Goodpasture's syndrome, acute glomerulonephritis, acute hypersensitivity intestinal nephritis, hypercalcemia, urinary tract obstruction, acute uric acid nephropathy, pyelonephritis, papillary necrosis, hepatorenal syndrome, hypertension, bacterial sepsis, systemic inflammatory shock, fungemia, acute viral diseases, severe body fluid volume depletion, burns, ischemia reperfusion, postpartum complications, surgical intervention, and drug nephritis, and is associated with renal ischemia or renal ischemia reperfusion.

13. A method of preparing a prophylactic or therapeutic agent for acute renal failure comprising glutathione trisulfide or a pharmacologically acceptable salt thereof, comprising steps of:
measuring a blood GDF-15 concentration in a subject; and
determining a content of glutathione trisulfide or a pharmacologically acceptable salt thereof in the prophylactic or therapeutic agent based on the blood GDF-15 concentration.

14. The method according to claim 13,
wherein the acute renal failure is acute renal failure resulting from at least one disease, disorder or condition selected from the group consisting of myoglobinuria rhabdomyolysis, hemoglobinuria, atheroembolism, renal artery occlusion, vasculitis, hemolytic uremic syndrome, thrombolytic thrombocytopenic purpura, Wegener's granulomatosis, systemic lupus erythematosus, Henoch-Schoenlein purpura, Goodpasture's syndrome, acute glomerulonephritis, acute hypersensitivity intestinal nephritis, hypercalcemia, urinary tract obstruction, acute uric acid nephropathy, pyelonephritis, papillary necrosis, hepatorenal syndrome, hypertension, bacterial sepsis, systemic inflammatory shock, fungemia, acute viral diseases, severe body fluid volume depletion, burns, ischemia reperfusion, postpartum complications, surgical intervention, and drug nephritis, and is associated with renal ischemia or renal ischemia reperfusion.

15. A method of measuring an indicator for prognosis determination and/or diagnosis of acute renal failure *in vitro*, comprising
measuring a GDF-15 concentration for a sample obtained from a subject as the indicator.

16. The method according to claim 15,
wherein the acute renal failure is acute renal failure resulting from at least one disease, disorder or condition selected from the group consisting of myoglobinuria rhabdomyolysis, hemoglobinuria, atheroembolism, renal artery occlusion, vasculitis, hemolytic uremic syndrome, thrombolytic thrombocytopenic purpura, Wegener's granulomatosis, systemic lupus erythematosus, Henoch-Schoenlein purpura, Goodpasture's syndrome, acute glomerulonephritis, acute hypersensitivity intestinal nephritis, hypercalcemia, urinary tract obstruction, acute uric acid nephropathy, pyelonephritis, papillary necrosis, hepatorenal syndrome, hypertension, bacterial sepsis, systemic inflammatory shock, fungemia, acute viral diseases, severe body fluid volume depletion, burns, ischemia reperfusion, postpartum complications, surgical intervention, and drug nephritis, and is associated with renal ischemia or renal ischemia reperfusion.
